# EUROPEAN PATENT SPECIFICATION

(11) **EP 3 061 446 B1**
(45) Date of publication and mention of the grant of the patent: **09.10.2019**
(21) Application number: 16157592.3
(22) Date of filing: 26.02.2016
(51) Int. Cl.: A61K 9/08, A61K 47/24, A61K 9/12, A61K 31/355, A61P 17/02

(54) **COMPOSITION CONTAINING VITAMIN E ACETATE FOR USE IN TOPICAL TREATMENT OF CRUSTS AND INFLAMMATION RESULTING FROM HAIR TRANSPLANTATION**
ZUSAMMENSETZUNG MIT VITAMIN E ACETAT ZUR VERWENDUNG IN DER TOPISCHEN BEHANDLUNG VON DURCH HAARTRANSPLANTATION VERURSACHTEN KRUSTEN UND ENTZÜNDUNG
COMPOSITION CONTENANT DE LA VITAMINE E ACÉTATE POUR UTILISATION DANS LE TRAITEMENT TOPIQUE DES CROÛTES ET DE L'INFLAMMATION SUITE À UNE TRANSPLANTATION DE CHEVEUX

(30) Priority: 26.02.2015 IT MI20150291
(43) Date of publication of application: 31.08.2016
(73) Proprietor: BIO.LO.GA. S.r.l., 31015 Conegliano (TV) (IT)
(72) Inventor: Panin, Giorgio, I-45100 Rovigo (IT); Navarro Belmonte, Maria Rogelia, 03004 Alicante (ES); Martínez Andrés, Maria Asunción, 03004 Alicante (ES); Molina Gil, Consuelo, 03010 Alicante (ES); Llorca Asín, Manuel, 03540 Alicante (ES); Martínez Serna, Ana Maria, 03670 Alicante (ES)
(74) Representative: Ferreccio, Rinaldo

(56) References cited:
- EP-A1- 0 998 943
- EP-A1- 2 233 124
- WO-A1-98/10793
- WO-A2-2010/031584
- US-A1- 2008 193 387
- JIMENEZ-ACOSTA F ET AL: "Follicular Unit Hair Transplantation: Current Technique", ACTAS DERMOSIFILIOGRAFICAS (ENGLISH EDITION), ELSEVIER, AMSTERDAM, NL, vol. 101, no. 4, 1 January 2010 (2010-01-01), pages 291-306, XP027580879, ISSN: 1578-2190 [retrieved on 2010-01-01]

## Description

### Field of application

The present invention relates to a composition containing vitamin E acetate for topical application for use in treating crusts and inflammation resulting from hair transplantation.

### Background art

Male and female alopecia may be a temporary hair loss or a premature and ultimate hair loss. There are various types of alopecia according to the causes that produce it or according to the form in which it occurs on the scalp. Androgenetic alopecia is the most common form of baldness (hereditary hair loss) and it is manifested by a progressive thinning of the hair with a tendency to become bald. The alopecia causes are various and sometimes fit together thus complicating the etiological framework and the definition of targeted local treatments. There may be genetic causes, responsible in particular for androgenetic alopecia or common baldness; hormonal causes, such as abnormal levels of testosterone, thyroid, pituitary or adrenal glands dysfunctions, food causes, such as lack of proteins, vitamins or minerals, anemia and anorexia, chemical and pharmacological causes, such as chemotherapy, antidepressants and other types of drugs. Finally, there are also psychological causes, such as traumatic shocks or depressive phenomena, and unnatural rhythms of work and life. In order to counteract alopecia, it is possible to intervene with hair care treatments, using vials and lotions to prevent the loss in order to contain the factors that cause hair loss or contribute to accelerate the death of hair follicles. Laser treatments are also being used to stimulate follicular activity promoting hair regrowth, and also high frequency energetic biostimulation treatments of hair follicles.

When the hair follicles are totally atrophied and insensitive to the above treatments, hair transplantation surgery is used.

The hair transplantation can be done with different microsurgical techniques, among which the most used are the F.U.S.S. (Follicular Unit Strip Surgery) technique and the F.U.E. (Follicular Unit Extraction) technique.

The F.U.S.S. technique involves the removal of a strip of scalp from the patient's nape (donor area), under local anesthesia, of a length of about 10 cm and 2 cm wide containing approximately 3.000-3.500 bulbs. After the withdrawal of the scalp from the occipital region, the sectioning of the strip is performed to extract the follicular units, which are then transplanted into the areas to be thickened.

The procedure involves a postoperative period which lasts about one month, since the closing of the withdrawn strip is made by means of a continuous suture which is normalized in 15-20 days. The disappearance of the crusts in the area of the hair implant is approximately 15-20 days (the incisions are more traumatic since realized with tools larger in size).

The F.U.E. technique is a hair transplant technique through direct withdrawal, without the withdrawal of the skin strip. In this technique, the various grafts (follicular units) are withdrawn one by one from the nape scalp, using a special cylindrical hollow scalpel (punch), and are directly and immediately transplanted in the affected portion to be thickened.

When using the F.U.E. technique, the extraction area of the scalp is shaved beforehand and then the isolated follicular units are obtained, that allows not to leave scars. The placement of the extracted follicular units is accomplished via an implanter of minimum size (snug fit) making small incisions, resulting in a considerable decrease in the patient's recovery time, which is 10-15 days.

A F.U.E. technique modified in the following aspects has been used since long time by the inventors of the present application:
1) the extraction zone is not shaved and this allows a more rapid reintegration of the subject in social life;
2) platelet rich plasma (PRP) is applied, infiltrating it by the mesotherapy technique in the donor and recipient areas, to minimize hair loss after surgery (postoperative telogenization), and the extracted grafts are preserved in PRP and under cooling, in order to enhance the yield at the time of transplantation.

A drawback of the F.U.E. technique is the formation of crusts and surgical micro wounds both in the extraction area and in the implant area, which are generally resolved in about 15-20 days, applying physiological solution both during surgery and in the postoperative period.

The presence of these crusts makes it difficult, if not impossible, the reintegration of the subject in the normal social life before the lapse of the said period.

The patent application WO 2010/031584 discloses the use of a composition containing vitamin E or a derivative thereof in a vehicle comprising a solvent selected from the group comprising volatile siloxanes, hydrogenated polyisobutene, hydrogenated polydecene and mixtures of hydrogenated polyisobutene, and/or polydecene hydrogenated with hydrogenated polyolefins, for the control of arthropods.

The patent application EP 2 233 124 discloses a textile support, for example a towel or a gauze, soaked in a cleanser/moisturizer composition comprising a volatile oil selected from volatile silicones, hydrogenated polyisobutene, hydrogenated polydecene and mixtures of hydrogenated polyisobutene, and/or hydrogenated polydecene with hydrogenated polyolefins and at least 10% by weight of a compound selected from vitamin E and esters thereof.

The patent application EP 0 998 943 discloses a formulation for topical use in the form of a hydrophobic gel, with moisturizing properties, comprising 20-70% by weight of vitamin E acetate, 20-70% of a volatile silicone and 7-13% of hydrogenated castor oil.

The patent application WO 98/10793 discloses a formulation for topical use, comprising a lipophilic phase which includes vitamin E or an ester thereof and may include silicone oils and volatile silicones.

US 2008/193387 discloses essential-oil compositions comprising Lippia Javanica essential oil in combination with other essential oils for killing or repelling ectoparasites, such as lice, and/or pests. Certain compositions also include tocopherol and cyclomethicone.

F. Jimenez-Acosta et al. "Follicular Unit Hair Transplantation: Current Technique", Actas Dermo-Sifiliograficas, 2010; 101(4):291-306 describes the formation of clots, crusts and inflammation as drawbacks in connection with hair transplantation procedures, but it does not disclose a composition for their treatment.

None of the documents mentioned above describes the use of compositions containing vitamin E or esters thereof for topical application during and after hair transplantation procedures.

The problem underlying the present invention was to speed up the resolution of the crusts resulting from the hair transplantation surgery, so to bring more quickly the hair scalp area affected by the surgery to a normal appearance and make an earlier reintegration of the subject in the social and work life, also keeping in good condition the hair shaft.

Such a problem has been solved according to the invention by means of a composition containing vitamin E acetate in a vehicle comprising a lipophilic solvent, wherein the lipophilic solvent is a volatile siloxane with a viscosity of less than 5·10⁻⁵ m²/s (50 centistokes), selected from pentamer cyclomethicone, tetramer cyclomethicone, hexamer cyclomethicone, hexamethyldisiloxane, dodecamethylpentasiloxane, dodecamethylcyclohexasiloxane and mixtures thereof, for use in treating crusts and inflammation resulting from hair transplantation.

It is particularly preferred the use of pentamer cyclomethicone.

In another preferred embodiment, the above-mentioned vehicle further comprises polydimethylsiloxane, preferably in an amount up to 50% b.w. of the weight of the vehicle. The presence of the polydimethylsiloxane enhances the combability and softness of the hair, which the composition is applied to.

Another particularly preferred vehicle is a mixture of dodecamethylpentasiloxane, dodecamethylcyclohexasiloxane and polydimethylsiloxane.

In an embodiment, the composition comprises from 1% to 90% of vitamin E acetate. Compositions suitable for the present invention comprise from 1 to 30% of vitamin E acetate.

According to one aspect of the present invention, a composition consisting essentially of vitamin E acetate and cyclomethicone, in particular cyclomethicone pentamer, is used.

According to another aspect of the present invention, a composition consisting essentially of vitamin E acetate, dodecamethylpentasiloxane, dodecamethylcyclohexasiloxane and polydimethylsiloxane is used.

Preferably, the composition that is used in the present invention is delivered by means of a spray delivery device.

Such a device for spray delivery can be constituted by a "gas-free" nebulizer or by a spray can containing the composition according to the invention and a propellant such as a saturated hydrocarbon, e.g. propane or butane, or a hydrofluorocarbon (HFC).

A "gas-free" spray device usable for the application of the composition used in the present invention has a container, in plastic or metal material containing the above described composition, on the entrance of which a valve with dip-tube is screwed or clamped, on which a dispenser operates.

According to one aspect of the present invention, the device for spray delivery of the composition comprises a collapsible container which is in turn contained in a spray can filled with a pressurized fluid, which causes said composition to be delivered in finely atomized form.

As pressurized fluid, saturated hydrocarbons, such as propane or butane, or fluorinated hydrocarbons (HFC) can be used but nitrogen, carbon dioxide and conveniently air are preferably used. The devices for spray delivery constituted by a can containing in its interior a collapsible container are described for example in the patent application FR 2 436 085. For the purposes of the present invention, the use of a spray device marketed by the company COSTER TECNOLOGIE SPECIALI SPA, ITALY has been resulted particularly convenient.

Using a spray device, it is possible to apply the composition in a finely atomized form, with the production of a film endowed with remarkable bioadhesivity characteristics in respect of the hair and of the scalp, which favors the action of the active ingredient.

Using the above described composition, it was possible to obtain a considerable acceleration of the resolution of the crusts generated on the scalp due to the hair transplant surgeries, obtaining a total resolution of such crusts within 7-10 days instead of 15-20 days necessary for the resolution of crusts when physiological solution, according to the known art, during and after surgery, is applied.

Furthermore, using the above mentioned composition in place of physiological solution during the surgical procedure, significant benefits have also been observed in keeping the hair shaft in good condition, and in the ability to disentangle the hair.

It has been observed, in particular, that the application of the above mentioned composition throughout the course of the surgery (lasting several hours) allows to easily lift and remove hair even in the case of female patients with long hair, because the used low viscosity vehicle allows to disentangle and separate the hair without weighing it down. Spray application also helps to facilitate the treatment of hair during the microsurgical procedure.

Furthermore, by applying the above mentioned composition during the surgery the formation of clots has been avoided, which in turn avoided that the inserts produced during the surgery "jumped" from their positions and permitted the formation of smaller and more quickly resolving crusts, allowing patient a rapid return to his social and working life.

With the use of the above mentioned composition according to the present invention, it was finally observed a less inflammation of the affected areas of the surgery, compared to the known use of physiological solution.

### Brief Description of Drawings

Further characteristics and advantages of the present invention will become more apparent from the following detailed description made with reference to the enclosed figures wherein:

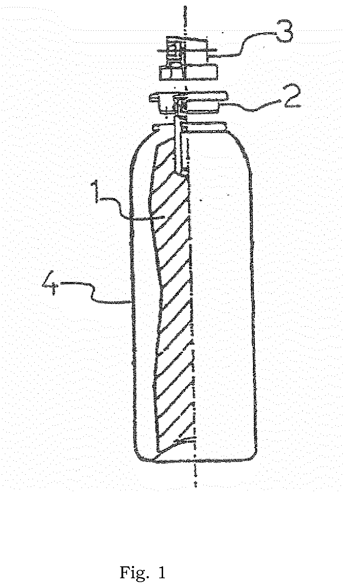

Figure 1 is a schematic representation of a spray device, usable for the application of the composition according to the present invention.
Figure 2 is a photograph of the scalp area in which a hair transplant has been carried out in a patient, using, according to the prior art, a physiological solution in the course of the surgical procedure, and subsequently to this procedure.
Figure 3 is a photograph of the scalp area in which a hair transplant has been carried out in a patient, using in the course of the surgical procedure, and after this procedure the composition according to the present invention.

### Detailed description

As mentioned above, the composition for use according to the present invention can be advantageously applied by means of a spray device.

A spray device usable for the application of the above mentioned composition has a collapsible container 1, containing the above described composition, connected to a valve 2, on which a dispenser 3 operates; the collapsible container 1 is in its turn enclosed within a can 4, with the valve 2 clamped to the edge of the can 4, and the space between the inner wall of the can 4 and the container 1 is filled with a pressurized gas.

### EXAMPLE 1

| | |
|---|---|
| d,l-alpha-tocopherol acetate | 20% |
| Cyclomethicone pentamer | 80% |

The above reported percentages, as well as those that will be described hereinafter, are percentages by weight on the total weight of the composition.

After having inserted the collapsible container 1 in the can 2 and clamped the valve 2 to the edge of the can 4, the space between the container 1 and the inner wall of the can 4 was filled with air at 10 atmospheres. At this point, the solution obtained by dispersing the alpha-tocopherol in cyclomethicone acetate was introduced under nitrogen flow into the collapsible container 1 according to methods known in the art.

### EXAMPLE 2

| | |
|---|---|
| d,l-alpha-tocopherol acetate | 10% |
| Cyclomethicone pentamer | 90% |

The solution obtained by dispersing the alpha-tocopherol acetate in the cyclomethicone was introduced into the collapsible container 1, as described in Example 1, and a spray device containing the above formulation was produced in the same manner as in Example 1.

### EXAMPLE 3

| | |
|---|---|
| d,l-alpha-tocopherol acetate | 5% |
| Cyclomethicone pentamer | 95% |

The solution obtained by dispersing the alpha-tocopherol acetate in the cyclomethicone was introduced into the collapsible container 1, as described in Example 1, and a spray device containing the above formulation was produced in the same manner as in Example 1.

### EXAMPLE 4

| | |
|---|---|
| d,l-alpha-tocopherol acetate | 1% |
| Cyclomethicone pentamer | 99% |

The solution obtained by dispersing the alpha-tocopherol acetate in the cyclomethicone was introduced into the collapsible container 1, as described in Example 1, and a spray device containing the above formulation was produced in the same manner as in Example 1.

### EXAMPLE 5

| | |
|---|---|
| d,l-alpha-tocopherol acetate | 15% |
| Cyclomethicone pentamer | 85% |

The solution obtained by dispersing the alpha-tocopherol acetate in the cyclomethicone was introduced into a plastic container, having an entrance, on which a valve with dip-tube was then screwed, on which a dispensing button operates.

### EXAMPLE 6

| | |
|---|---|
| d,l-alpha-tocopherol acetate | 20% |
| Belsil DM2 | 80% |

The solution obtained by dispersing the alpha-tocopherol acetate in the Belsil DM2 (which is a mixture of dodecamethylpentasiloxane, dodecamethylcyclohexasiloxane and polydimethylsiloxane sold by the firm Wacker) was introduced into the collapsible container 1, as described in Example 1, and a spray device containing the above formulation was produced in the same manner as in Example 1.

The inventors of the present patent application have used the composition according to Example 1, in spray form, marketed with the name of VEA® SPRAY by the company HULKA S.r.1. from Rovigo (IT) in hair transplant surgeries according to F.U.E. technique on over 300 patients.

A VEA® SPRAY application protocol was followed during the surgical process, using the spray both in the extraction area and in the implant area of the inserts, as a microsurgery adjuvant, achieving substantial benefits in the resolution of the crusts, in the maintenance of the hair shaft and in the ability to disentangle the hair.

According to such a protocol, once the surgery was over, with the scalp already impregnated with VEA® SPRAY, the hair was washed with mild shampoo and after washing VEA® SPRAY was still applied in the same area and then the hair was dried, which was washed again the next day. The protocol also provides that the patient, once discharged, continues to apply at home VEA® SPRAY 2 times a day in the donor area and in the recipient area, in the morning half-hour before washing the hair and the night before going to sleep, repeating the treatment for 10 days.

In all patients treated with the above mentioned protocol a total resolution of the crusts in 7-10 days was observed, with a tolerance of SPRAY VEA® product of 100%, observing a cosmetic benefit particularly pronounced in female patients.

In respect of the protocols previously adopted, which included the use of physiological solution instead of the product VEA® SPRAY, surprisingly superior results were achieved in terms of maintaining the good condition of the hair shaft and especially in the acceleration of the resolution of the crusts after surgery, being these less numerous and important from the first day until their resolution in 7-10 days, instead of the 15-20 days duration of crusts observed with the use of physiological solution.

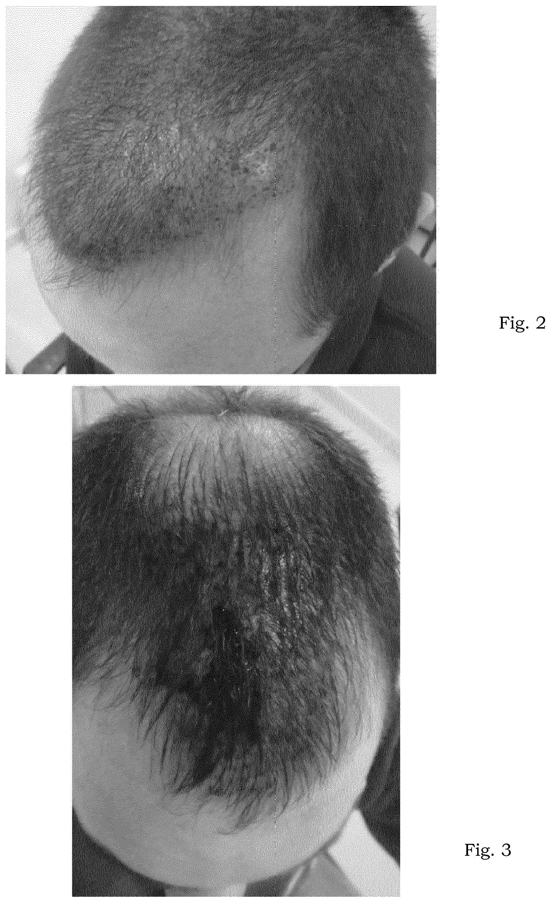

From the photograph of Figure 3, taken the day after the transplant surgery with F.U.E technique, it was noted that the use of VEA® SPRAY product during surgery and in the days after that surgery led to the formation of crusts of reduced size and which appear already in the process of resolution.

The photograph of Figure 2, also taken the day after the transplant surgery with F.U.E technique, shows that the use of physiological solution during the surgery and in the days after that surgery led to the formation of crusts of large size still evident.

The inventors of the present application have also used an implanter of an adapted size (snug fit), by which they have obtained that the graft once implanted would not be lost, that there was a maximum contact of the implanted hair with its bed and that consequently the oxygenation was quickly re-established and that the elimination of dead spaces was achieved. Furthermore, the use of the said implanter in combination with the application of the SPRAY VEA® product has reduced the formation of clots and has facilitated the healing of surgical wounds. The direct consequence of the failure to formation of large crusts has been the reduction of the time needed to fix the inserts up to a maximum of 4 days.

In particular, the absence of the formation of clots is in large part attributable to the use of the VEA® SPRAY product, since such absence of clots was not observed with the use of physiological solution. As a result of this lack of clot formation it was obtained that the inserts did not "jump" from their positions and that smaller crusts and a faster resolution were achieved, thus allowing a rapid return for patient to his social and working life.

From preliminary clinical experiences made on small groups of patients of both genders it was showed that the favorable results obtained with the use of the SPRAY VEA® product were also obtained, in comparable extent, with the use of the spray formulations according to Examples 2-7.

## Claims

1. A composition containing vitamin E acetate in a vehicle comprising a lipophilic solvent, wherein said lipophilic solvent is a volatile siloxane having a viscosity of less than 5·10⁻⁵ m²/s (50 centistokes) measured at 25 °C selected from pentamer cyclomethicone, tetramer cyclomethicone, hexamer cyclomethicone, hexamethyldisiloxane, dodecamethylpentasiloxane, dodecamethylcyclohexasiloxane and mixtures thereof, for use in treating crusts and inflammation resulting from hair transplantation.

2. The composition for the use according to claim 1, wherein said vehicle further comprises polydimethylsiloxane.

3. The composition for the use according to claim 2, wherein said vehicle comprises dodecamethylpentasiloxane, dodecamethylcyclohexasiloxane and polydimethylsiloxane.

4. The composition for the use according to anyone of claims 1-3, wherein said composition comprises from 1% to 90%, preferably from 1 to 30% of vitamin E acetate.

5. The composition for the use according to anyone of claims 1-4, wherein said composition consists essentially of vitamin E acetate and cyclomethicone, preferably pentamer cyclomethicone.

6. The composition for the use according to claim 3, wherein said composition consists essentially of vitamin E acetate, dodecamethylpentasiloxane, dodecamethylcyclohexasiloxane and polydimethylsiloxane.

7. The composition for the use according to claim 5 or 6, wherein said composition comprises from 1% to 90%, preferably from 1 to 30% of vitamin E acetate.

8. The composition for the use according to anyone of claims 1-7, wherein said composition is delivered by means of a spray delivery device.

9. The composition for the use according to claim 8, wherein said device comprises a collapsible container (1), which is in turn contained in a spray can (4) filled with a pressurized fluid, which causes said composition to be delivered in finely atomised form.

## Patentansprüche

1. Eine Zusammensetzung, die Vitamin E-Acetat in einem Träger enthält, umfassend ein lipophiles Lösungsmittel, wobei das lipophile Lösungsmittel ein flüchtiges Siloxan mit einer Viskosität von weniger als 5·10⁻⁵ m²/s (50 Centistokes) ist, gemessen bei 25 °C, ausgewählt aus Pentamercyclomethicon, Tetramercyclomethicon, Hexamercyclomethicon, Hexamethyldisiloxan, Dodecamethylpentasiloxan, Dodecamethylcyclohexasiloxan und Mischungen davon, zur Verwendung bei der Behandlung von durch Haartransplantation verursachten Krusten und Entzündung.

2. Die Zusammensetzung zur Verwendung nach Anspruch 1, wobei der Träger weiter Polymethylsiloxan umfasst.

3. Die Zusammensetzung zur Verwendung nach Anspruch 2, wobei der Träger Dodecamethylpentasiloxan, Dodecamethylcyclohexasiloxan, und Polydimethylsiloxan umfasst.

4. Die Zusammensetzung zur Verwendung nach einem der vorhergehenden Ansprüche 1-3, wobei die Zusammensetzung 1% bis 90%, vorzugsweise 1% bis 30% Vitamin E-Acetat umfasst.

5. Die Zusammensetzung zur Verwendung nach einem der vorhergehenden Ansprüche 1-4, wobei die Zusammensetzung im Wesentlichen aus Vitamin E-Acetat und Cyclomethicon, vorzugsweise Pentamercyclomethicon, besteht.

6. Die Zusammensetzung zur Verwendung nach Anspruch 3, wobei die Zusammensetzung im Wesentlichen aus Vitamin E-Acetat, Dodecamethylpentasiloxan, Dodecamethylcyclohexasiloxan und Polydimethylsiloxan besteht.

7. Die Zusammensetzung zur Verwendung nach Anspruch 5 oder 6, wobei die Zusammensetzung 1% bis 90%, vorzugsweise 1% bis 30% Vitamin E-Acetat umfasst.

8. Die Zusammensetzung zur Verwendung nach einem der vorhergehenden Ansprüche 1-7, wobei die Zusammensetzung durch eine Sprüheinrichtung abgegeben wird.

9. Die Zusammensetzung zur Verwendung nach Anspruch 8, wobei die Sprüheinrichtung einen komprimierbaren Behälter (1) umfasst, der in einer mit einem unter Druck stehenden Fluid gefüllten Sprühdose (4) enthalten ist, sodass die Zusammensetzung in fein zerstäubter Form abgegeben wird.

## Revendications

1. Composition contenant de l'acétate de vitamine E dans un véhicule comprenant un solvant lipophile, dans laquelle ledit solvant lipophile est un siloxane volatil ayant une viscosité inférieure à 5·10⁻⁵ m²/s (50 centistokes) mesurée à 25 °C sélectionné parmi la cyclométhicone pentamère, la cyclométhicone tétramère, la cyclométhicone hexamère, l'hexaméthyldisiloxane, le dodécaméthylpentasiloxane, le dodécaméthylcyclohexasiloxane et les mélanges de ceux-ci, pour son utilisation dans le traitement des croûtes et d'une inflammation résultant de la transplantation de cheveux.

2. Composition pour son utilisation selon la revendication 1, dans laquelle ledit véhicule comprend en outre du polydiméthylsiloxane.

3. Composition pour son utilisation selon la revendication 2, dans laquelle ledit véhicule comprend du dodécaméthylpentasiloxane, du dodécaméthylcyclohexasiloxane et du polydiméthylsiloxane.

4. Composition pour son utilisation selon l'une quelconque des revendications 1 à 3, dans laquelle ladite composition comprend de 1 % à 90 %, de préférence de 1 à 30 % d'acétate de vitamine E.

5. Composition pour son utilisation selon l'une quelconque des revendications 1 à 4, dans laquelle ladite composition est essentiellement constituée d'acétate de vitamine E et de cyclométhicone, de préférence de cyclométhicone pentamère.

6. Composition pour son utilisation selon la revendication 3, dans laquelle ladite composition est essentiellement constituée d'acétate de vitamine E, de dodécaméthylpentasiloxane, de dodécaméthylcyclohexasiloxane et de polydiméthylsiloxane.

7. Composition pour son utilisation selon la revendication 5 ou 6, dans laquelle ladite composition comprend de 1 % à 90 %, de préférence de 1 à 30 % d'acétate de vitamine E.

8. Composition pour son utilisation selon l'une quelconque des revendications 1 à 7, dans laquelle ladite composition est administrée au moyen d'un dispositif d'administration par pulvérisation.

9. Composition pour son utilisation selon la revendication 8, dans laquelle ledit dispositif comprend un récipient compressible (1), qui est à son tour contenu dans une bombe aérosol (4) remplie d'un fluide pressurisé, qui entraîne l'administration de ladite composition sous une forme finement atomisée.
